# EUROPEAN PATENT APPLICATION

(11) **EP 4 582 562 A1**
(43) Date of publication of application: **09.07.2025**
(21) Application number: 23860515.8
(22) Date of filing: 31.08.2023
(51) Int. Cl.: C12Q 1/6886, C12N 15/11, C12Q 1/6827, G01N 33/574

(54) **METHOD, POLYNUCLEOTIDE, AND KIT FOR ASSESSING RISK OF ONSET OF STOMACH CANCER IN PATIENT AFTER REMOVING HELICOBACTER PYLORI**

(30) Priority: 02.09.2022 JP 2022140097
(71) Applicant: The Kitasato Institute, Tokyo 108-8641 (JP)
(72) Inventor: FUKUYAMA Takashi, Sagamihara-shi, Kanagawa 252-0373 (JP); KOBAYASHI Noritada, Sagamihara-shi, Kanagawa 252-0373 (JP); YAMAZAKI Taiga, Sagamihara-shi, Kanagawa 252-0373 (JP); TAKAHASHI Yoshihito, Sagamihara-shi, Kanagawa 252-0373 (JP); YAMAZAKI Hitoshi, Sagamihara-shi, Kanagawa 252-0373 (JP); FUTAWATARI Nobue, Tokyo 153-8515 (JP); OTSUKA Toshikazu, Sagamihara-shi, Kanagawa 252-0373 (JP); KOIZUMI Wasaburo, Sagamihara-shi, Kanagawa 252-0373 (JP); ICHIKI Yoshinobu, Wako-shi, Saitama 351-0102 (JP); SAKAGUCHI Kanako, Sagamihara-shi, Kanagawa 252-0373 (JP)
(74) Representative: Regimbeau
(86) International application number: PCT/JP2023/031975
(87) International publication number: WO 2024/048753

(57) **Abstract**

A method for assessing a risk of onset of stomach cancer in a patient after removing Helicobacter pylori, the method including: a step of detecting expression of a KK-LC-1 gene in a stomach tissue sample derived from the patient before removing Helicobacter pylori, in which positive expression of the KK-LC-1 gene indicates that there is a risk of onset of stomach cancer in the patient after removing Helicobacter pylori.

## Description

### TECHNICAL FIELD

The present invention relates to a method, a polynucleotide, and a kit for assessing a risk of onset of stomach cancer in a patient after removing Helicobacter pylori. Priority is claimed on Japanese Patent Application No. 2022-140097, filed September 2, 2022, the content of which is incorporated herein by reference.

### BACKGROUND ART

It is known that Helicobacter pylori infection is one of the causes of stomach cancer onset. Currently, in a case where Helicobacter pylori infection is found, the removal of Helicobacter pylori by oral administration is covered by health insurance. It has been reported that the risk of onset of stomach cancer in a case in which Helicobacter pylori is removed is reduced to 1/3 compared to a non-removal group. On the other hand, this result indicates that there are cases in which cancer occurs even when Helicobacter pylori is removed. At present, there is no technology for assessing the risk of onset of stomach cancer after removing Helicobacter pylori. That is, even in a case in which the removal is successful, the target patient has continuous anxiety about cancer occurrence as in a case before the removal. Further, since there is no risk index of cancer occurrence after the removal, regular medical checkups are required for all successful removal cases, and the medical costs or medical resources are not reduced. The risk index as described above is an unmet medical need that is strongly required from the medical field and the patient side.

Incidentally, cancer/testis antigens are a general term for proteins whose expression is not observed in any tissue other than cancer cells and the testis. Kitakyushu lung cancer antigen-1 (KK-LC-1) is one type of cancer/testis antigen. The present inventors have found that KK-LC-1 can be used as a marker expressed in various cancers (for example, refer to Patent Document 1).

### Citation List

### Patent Document

Patent Document 1: Japanese Patent No. 6028253

### SUMMARY OF INVENTION

### Technical Problem

An object of the present invention is to provide a technology for assessing a risk of onset of stomach cancer in a patient after removing Helicobacter pylori.

### Solution to problem

The present invention includes the following aspects.
[1] A method for assessing a risk of onset of stomach cancer in a patient after removing Helicobacter pylori, the method including: a step of detecting expression of a KK-LC-1 gene in a stomach tissue sample derived from the patient before removing Helicobacter pylori, in which positive expression of the KK-LC-1 gene indicates that there is a risk of onset of stomach cancer in the patient after removing Helicobacter pylori.
[2] The method according to [1], in which the detecting of the expression of the KK-LC-1 gene is performed by end-point RT-PCR.
[3] The method according to [2], in which in the end-point RT-PCR, a primer consisting of a base sequence set forth in SEQ ID NO: 1 and a primer consisting of a base sequence set forth in SEQ ID NO: 2 are used.
[4] The method according to [1], in which the detecting of the expression of the KK-LC-1 gene is performed by real-time RT-PCR.
[5] The method according to [4], in which in the real-time RT-PCR, a qPCR probe is used, and the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.
[6] The method according to [4] or [5], in which the real-time RT-PCR is performed in presence of a single-stranded nucleic acid fragment that suppresses amplification of genomic DNA, and the single-stranded nucleic acid fragment has a base sequence including 322nd and 323rd bases or 1096th and 1097th bases in a base sequence set forth in SEQ ID NO: 4, and has a base length of 20 to 50 bases.
[7] The method according to [1], in which the detecting of the expression of the KK-LC-1 gene is performed at a protein level.
[8] A kit for assessing a risk of onset of stomach cancer in a patient after removing Helicobacter pylori, the kit including: a primer consisting of a base sequence set forth in SEQ ID NO: 1; and a primer consisting of a base sequence set forth in SEQ ID NO: 2.
[9] The kit according to [8], further including: a qPCR probe, in which the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.
[10] The kit according to [8] or [9], further including: a single-stranded nucleic acid fragment that suppresses amplification of genomic DNA, in which the single-stranded nucleic acid fragment has a base sequence including 322nd and 323rd bases or 1096th and 1097th bases in a base sequence set forth in SEQ ID NO: 4, and has a base length of 20 to 50 bases.
[11] A kit for assessing a risk of onset of stomach cancer in a patient after removing Helicobacter pylori, the kit including: a specific binding substance to a KK-LC-1 protein.
[12] A polynucleotide having a base sequence including 195th and 196th bases in a base sequence set forth in SEQ ID NO: 3, having a base length of 15 to 50 bases, and having a one- or two-base mutation with respect to the base sequence set forth in SEQ ID NO: 3.
[13] A polynucleotide having a base sequence including 322nd and 323rd bases or 1096th and 1097th bases in a base sequence set forth in SEQ ID NO: 4, and having a base length of 20 to 50 bases.
[14] A method for detecting expression of a KK-LC-1 gene in a biological sample, in which the detecting is performed by real-time RT-PCR, in the real-time RT-PCR, a qPCR probe is used, the real-time RT-PCR is performed in presence of a single-stranded nucleic acid fragment that suppresses amplification of genomic DNA, and the single-stranded nucleic acid fragment has a base sequence including 322nd and 323rd bases or 1096th and 1097th bases in a base sequence set forth in SEQ ID NO: 4, and has a base length of 20 to 50 bases.
[15] The method according to [14], in which the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.

### Advantageous Effects of Invention

According to the present invention, a technology for assessing a risk of onset of stomach cancer in a patient after removing Helicobacter pylori can be provided.

### BRIEF DESCRIPTION OF DRAWINGS

[FIG. 1] A graph showing a 5-year cumulative stomach cancer onset rate in patients in a KK-LC-1 positive group and a negative group in Experimental Example 1.
[FIG. 2] A graph showing a 5-year cumulative stomach cancer onset rate in patients in a KK-LC-1 positive group and a negative group in Experimental Example 2.
[FIG. 3] A graph showing the results of real-time PCR in Experimental Example 3.
[FIG. 4] A graph showing the results of real-time PCR in Experimental Example 3.
[FIG. 5] A graph showing the results of real-time PCR in Experimental Example 3.
[FIG. 6] A graph showing the results of real-time PCR in Experimental Example 3.
[FIG. 7] A graph showing the results of real-time PCR in Experimental Example 4.
[FIG. 8] A graph showing the results of real-time PCR in Experimental Example 4.
[FIG. 9] A graph showing the results of the real-time PCR in Experimental Example 5.
[FIG. 10] A graph showing the results of real-time PCR in Experimental Example 6.
[FIG. 11] A graph showing the results of real-time PCR in Experimental Example 6.

### DESCRIPTION OF EMBODIMENTS

### [Method for Assessing Risk of Onset of Stomach Cancer in Patient After Removing Helicobacter Pylori]

In one embodiment, the present invention provides a method for assessing a risk of onset of stomach cancer in a patient after removing Helicobacter pylori, the method including a step of detecting expression of a KK-LC-1 gene in a stomach tissue sample derived from the patient before removing Helicobacter pylori, in which positive expression of the KK-LC-1 gene indicates that there is a risk of onset of stomach cancer in the patient after removing Helicobacter pylori.

The inventors have found that in a stomach tissue sample derived from a patient before removing Helicobacter pylori, in a case where the expression of the KK-LC-1 gene is positive, there is a risk of onset of stomach cancer in the patient after removing Helicobacter pylori, and have completed the present invention. The NCBI accession number of the cDNA of the human KK-LC-1 gene is NM_001017978.4. In addition, the NCBI accession number of the genomic DNA of the human KK-LC-1 gene is NC_000023.11. In addition, the NCBI accession number of the human KK-LC-1 protein is NP_001017978.1.

As described later in Examples, according to the method according to the present embodiment, the risk of onset of stomach cancer in a patient after removing Helicobacter pylori can be assessed. In the related art, there is no technology for assessing the risk of onset of stomach cancer after removing Helicobacter pylori.

As the stomach tissue sample, a stomach tissue sample collected at the time of confirming the infection with Helicobacter pylori can be suitably used. In a case where the expression of the KK-LC-1 gene is positive, this case indicates that there is a risk of onset of stomach cancer in a patient even after removing Helicobacter pylori. In this case, measures such as increasing the frequency of examining the patient's stomach cancer can be taken.

In a case where the expression of the KK-LC-1 gene is negative, this case indicates that there is a low risk of onset of stomach cancer in a patient after removing Helicobacter pylori. In this case, the frequency of examining the patient's stomach cancer can be reduced, and limited medical resources can be saved. In addition, the anxiety of the patient who is worried about the onset of stomach cancer can be reduced and the psychological burden of the patient can be reduced.

In the method of the present embodiment, the detection of the expression of the KK-LC-1 gene may be performed by end-point RT-PCR. In this case, it is preferable to use a primer consisting of a base sequence set forth in SEQ ID NO: 1 and a primer consisting of a base sequence set forth in SEQ ID NO: 2. Here, the end-point RT-PCR means general RT-PCR which is not real-time RT-PCR.

In the method of the present embodiment, the detection of the expression of the KK-LC-1 gene may be performed by real-time RT-PCR. As described later in Examples, by detecting the expression of the KK-LC-1 gene by real-time RT-PCR, it is possible to more accurately assess the risk of onset of stomach cancer in the patient after removing Helicobacter pylori as compared with the end-point RT-PCR.

In the real-time RT-PCR, it is preferable to use a qPCR probe. The qPCR probe is also referred to as a TaqMan (registered trademark) probe, and is an oligonucleotide modified with a fluorescent substance and a quencher substance. The qPCR probe specifically hybridizes to template DNA in an annealing step of the PCR reaction. Since the qPCR probe has a quencher substance on the probe, the generation of fluorescence is suppressed even when the probe is irradiated with excitation light.

In the subsequent extension reaction step, when the qPCR probe hybridized to the template is degraded by the 5' to 3' exonuclease activity of the Taq DNA polymerase. As a result, the fluorescent substance and the quencher substance are liberated, and fluorescence is generated as a result of release from suppression by the quencher substance.

In the real-time RT-PCR using the qPCR probe, the quantification of the nucleic acid is performed by using a product amplified by PCR, that is, linearity that is obtained between the number of cycles when the fluorescence intensity reaches a certain level, and the initial amount of the target DNA.

In the real-time RT-PCR, for example, the Ct value of β-actin may be used as a reference, and a difference between the Ct value of β-actin and the Ct value of the KK-LC-1 gene may be obtained and used as ΔCt. In this case, for example, a case where ΔCt is -2 or more may be classified as a high expression group, a case where ΔCt is -5 or more and less than -2 may be classified as a medium expression group, and a case where ΔCt is less than -5 may be classified as a low expression group.

The NCBI accession number of the cDNA of the human β-actin gene is NM_001101.5. In addition, the NCBI accession number of the human β-actin protein is NP_001092.1.

As described later in Examples, the 5-year cumulative stomach cancer onset rate was 40% in the KK-LC-1 high expression group, and the 5-year cumulative stomach cancer onset rate was 5% in the medium expression group. On the other hand, in the KK-LC-1 low expression group, the 5-year cumulative stomach cancer onset rate was 0%. Therefore, it can be said that in a case of using the Ct value of β-actin as a reference, the smaller the ΔCt, the lower the risk of the onset of stomach cancer in the patient after removing Helicobacter pylori.

In the method of the present embodiment, it is preferable that the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.

The genomic DNA encoding KK-LC-1 has a first exon, an intron, and a second exon. The base sequence of the genomic DNA of KK-LC-1 is shown in SEQ ID NO: 4.

The base sequence set forth in SEQ ID NO: 3 is the base sequence of cDNA of the KK-LC-1 gene. The 195th base in SEQ ID NO: 3 corresponds to the last base of the first exon of KK-LC-1. In addition, the 196th base in SEQ ID NO: 3 corresponds to the first base of the second exon of KK-LC-1.

In the method of the present embodiment, the qPCR probe preferably has a base sequence including the 195th and 196th bases in the base sequence set forth in SEQ ID NO: 3. That is, the qPCR probe preferably hybridizes to a linking site between the first exon and the second exon in the cDNA of the KK-LC-1 gene.

Such a qPCR probe is a probe in which while the hybridization ability to the cDNA of the KK-LC-1 gene is maintained, the hybridization to the genomic DNA of the KK-LC-1 gene is reduced. As a result, it is possible to more accurately detect the cDNA of the KK-LC-1 gene.

In addition, the base length of the qPCR probe is not particularly limited as long as the Tm value of the qPCR probe can be appropriately set, may be about 15 to 50 bases, for example, may be 15 to 40 bases, and for example, may be about 15 to 30 bases.

The qPCR probe may be modified with a minor groove binder (MGB). The Tm value can be increased by modification with MGB. Therefore, in a case where the qPCR probe is modified with MGB, it is also possible to design the base length of the qPCR probe to be shorter.

The MGB is not particularly limited as long as the Tm value is increased, and examples thereof include dihydropyrroloindole tripeptide (CDPI3), Hoechst 33258, and the like.

In addition, in the method of the present embodiment, the qPCR probe preferably has a one- or two-base mutation with respect to the base sequence (SEQ ID NO: 3) of the cDNA of the KK-LC-1 gene.

There are similar portions in the base sequence of the intron and the base sequence of the second exon of the KK-LC-1 gene. Therefore, the qPCR probe that hybridizes to the linking site between the first exon and the second exon in the cDNA of the KK-LC-1 gene may also hybridize to the genomic DNA of the KK-LC-1 gene.

On the other hand, as described later in Examples, when the qPCR probe has a one- or two-base mutation with respect to the base sequence (SEQ ID NO: 3) of the cDNA of the KK-LC-1 gene, the hybridization to the genomic DNA of the KK-LC-1 gene is reduced while the hybridization ability to the cDNA of the KK-LC-1 gene is maintained. As a result, it is possible to more accurately detect the cDNA of the KK-LC-1 gene. Examples of such a qPCR probe include qPCR probes having a base sequence set forth in any of SEQ ID NOs: 5 to 10. Among these, a qPCR probe having a base sequence set forth in any of SEQ ID NOs: 7 to 10 is preferable.

In the method of the present embodiment, the above-described real-time RT-PCR can also be performed in the presence of a single-stranded nucleic acid fragment that suppresses amplification of genomic DNA. It is preferable that the single-stranded nucleic acid fragment has a base sequence including 322nd and 323rd bases or 1096th and 1097th bases in the base sequence (SEQ ID NO: 4) of the genomic DNA of KK-LC-1, and has a base length of 20 to 50 bases. The base length of the single-stranded nucleic acid fragment is not particularly limited as long as the Tm value of the single-stranded nucleic acid fragment can be appropriately set, may be about 20 to 50 bases, for example, may be 20 to 40 bases, for example, may be 25 to 35 bases, for example, may be 20 to 34 bases, and for example, may be 20 to 31 bases.

The 1096th base in SEQ ID NO: 4 corresponds to the first base of the intron of KK-LC-1. In addition, the 1097th base in SEQ ID NO: 4 corresponds to the last base of the first exon of KK-LC-1.

Therefore, the single-stranded nucleic acid fragment having the base sequence including the 1096th and 1097th bases in SEQ ID NO: 4 can hybridize to the linking site between the first exon and the intron of the genomic DNA of KK-LC-1 and can prevent the qPCR probe from hybridizing to the genomic DNA of KK-LC-1. As a result, it is possible to more accurately detect the cDNA of the KK-LC-1 gene.

The 322nd base in SEQ ID NO: 4 corresponds to the first base of the second exon of KK-LC-1. In addition, the 323rd base in SEQ ID NO: 4 corresponds to the last base of the intron of KK-LC-1.

Therefore, the single-stranded nucleic acid fragment having the base sequence including the 322nd and 323rd bases in SEQ ID NO: 4 can hybridize to the linking site between the intron and the second exon of the genomic DNA of KK-LC-1 and can prevent the qPCR probe from hybridizing to the genomic DNA of KK-LC-1. As a result, it is possible to more accurately detect the cDNA of the KK-LC-1 gene. Examples of such a single-stranded nucleic acid fragment include single-stranded nucleic acid fragments having a base sequence set forth in any of SEQ ID NOs: 11 to 13.

In the method of the present embodiment, the detection of the expression of the KK-LC-1 gene may be performed at a protein level. In this case, the KK-LC-1 protein in the stomach tissue sample may be detected by ELISA, Western blotting, or the like using a specific binding substance to KK-LC-1. The specific binding substance will be described later.

### [Kit for Assessing Risk of Onset of Stomach Cancer in Patient After Removing Helicobacter Pylori]

In one embodiment, the present invention provides a kit for assessing a risk of onset of stomach cancer in a patient after removing Helicobacter pylori, the kit including a primer consisting of a base sequence set forth in SEQ ID NO: 1; and a primer consisting of a base sequence set forth in SEQ ID NO: 2.

The kit of the present embodiment may further include a qPCR probe, and the qPCR probe may have a base sequence including 195th and 196th bases in a base sequence set forth in SEQ ID NO: 3, may have a base length of 15 to 50 bases, and may have a one- or two-base mutation with respect to the base sequence set forth in SEQ ID NO: 3. The qPCR probe is the same as described above.

The kit according to the present embodiment may further include a single-stranded nucleic acid fragment that suppresses amplification of genomic DNA, and the single-stranded nucleic acid fragment may have a base sequence including 322nd and 323rd bases or 1096th and 1097th bases in a base sequence set forth in SEQ ID NO: 4, and may have a base length of 20 to 50 bases. The single-stranded nucleic acid fragment that suppresses amplification of genomic DNA is the same as described above.

The kit of the present embodiment may include a specific binding substance to the KK-LC-1 protein. Examples of the specific binding substance to the KK-LC-1 protein include antibodies to KK-LC-1 and aptamers to KK-LC-1. The antibody may be an antibody fragment. Examples of the antibody fragment include F(ab')₂, Fab', Fab, Fv, and scFv. In addition, examples of the aptamers include nucleic acid aptamers, and peptide aptamers.

Using these kits, the above-described method for assessing the risk of onset of stomach cancer can be suitably carried out.

### [Polynucleotide]

In one embodiment, the present invention provides a polynucleotide having a base sequence including 195th and 196th bases in the base sequence set forth in SEQ ID NO: 3, having a base length of 15 to 50 bases, and having a one- or two-base mutation with respect to the base sequence set forth in SEQ ID NO: 3.

It is preferable that the polynucleotide of the present embodiment is modified with a fluorescent substance and a quencher substance to be configured as a qPCR probe.

As the fluorescent substance, a fluorescent substance that is generally used for a qPCR probe can be used without particular limitation. Specific examples of the fluorescent substance include fluorescein, Alexa 488, ATTO 542, Alexa 647, FAM, Cy5, and Cy3.

As the quencher substance, a substance that is generally used for a qPCR probe can be used without particular limitation. Specific examples of the quencher substance include Black Hole Quencher (BHQ) (registered trademark)-1, BHQ (registered trademark)-2, BHQ (registered trademark)-3, Iowa Black FQ, and Iowa Black RQ. As the quencher substance, a quencher substance capable of quenching the fluorescence of the fluorescent substance to be used is selected.

The polynucleotide of the present embodiment can be suitably used as the above-described qPCR probe. By using the polynucleotide of the present embodiment as the qPCR probe and performing the real-time RT-PCR, it is possible to more accurately detect the cDNA of the KK-LC-1 gene.

In another embodiment, the present invention provides a polynucleotide having a base sequence including 322nd and 323rd bases or 1096th and 1097th bases in the base sequence set forth in SEQ ID NO: 4, and having a base length of 20 to 50 bases. The polynucleotide of the present embodiment can be suitably used as the single-stranded nucleic acid fragment described above, which suppresses amplification of genomic DNA.

More specific examples of the single-stranded nucleic acid fragment include single-stranded nucleic acid fragments having a base sequence set forth in any of SEQ ID NOs: 11 to 13. As described above, by performing the real-time RT-PCR in the presence of the single-stranded nucleic acid fragment according to the present embodiment, it is possible to more accurately detect the cDNA of the KK-LC-1 gene.

### [Method for Detecting Expression of KK-LC-1 Gene in Biological Sample]

In one embodiment, the present invention provides a method for detecting expression of a KK-LC-1 gene in a biological sample, in which the detecting is performed by real-time RT-PCR, in the real-time RT-PCR, a qPCR probe is used, the real-time RT-PCR is performed in presence of a single-stranded nucleic acid fragment that suppresses amplification of genomic DNA, and the single-stranded nucleic acid fragment has a base sequence including 322nd and 323rd bases or 1096th and 1097th bases in a base sequence set forth in SEQ ID NO: 4, and has a base length of 20 to 50 bases. The single-stranded nucleic acid fragment is the same as described above.

As described above, the single-stranded nucleic acid fragment can hybridize to the linking site between the first exon and the intron of genomic DNA of KK-LC-1 and can prevent the qPCR probe from hybridizing to the genomic DNA of KK-LC-1. As a result, as described later in Examples, it is possible to more accurately detect the cDNA of the KK-LC-1 gene. Here, the fact that the detection can be more accurately performed means that the detection of false positive is suppressed as compared with a case where the above-described single-stranded nucleic acid fragment is not used.

In the method according to the present embodiment, examples of the biological sample include tissues, serum, blood plasmas, cultured cells, and cell culture supernatants derived from humans or non-human animals.

In the method of the present embodiment, it is preferable that the qPCR probe has the base sequence including the 195th and 196th bases in the base sequence set forth in SEQ ID NO: 3, has a base length of 15 to 50 bases, and has a one- or two-base mutation with respect to the base sequence set forth in SEQ ID NO: 3. As described above, such a qPCR probe is a probe in which while the hybridization ability to the cDNA of the KK-LC-1 gene is maintained, the hybridization to the genomic DNA of the KK-LC-1 gene is reduced. As a result, it is possible to more accurately detect the cDNA of the KK-LC-1 gene. As described above, examples of such a qPCR probe include qPCR probes having a base sequence set forth in any of SEQ ID NOs: 5 to 10. Among these, a qPCR probe having a base sequence set forth in any of SEQ ID NOs: 7 to 10 is preferable.

### Examples

Hereinafter, the present invention will be described in more detail by way of Examples; however, the present invention is not intended to be limited to the following Examples.

### [Experimental Example 1]

### (Study by End-Point RT-PCR)

In cases of 111 patients after removing Helicobacter pylori, stomach tissue samples collected before the removal were subjected to RT-PCR, and the expression of the KK-LC-1 gene was detected.

In the RT-PCR, a primer consisting of a base sequence set forth in SEQ ID NO: 1 and a primer consisting of a base sequence set forth in SEQ ID NO: 2 were used. After 40 cycles of PCR reaction, agarose gel electrophoresis was performed, a case where a visible band was detected was determined to be positive, and a case where a visible band was not detected was determined to be negative. In addition, the onset of stomach cancer in each patient after Helicobacter pylori was removed was followed up for 5 years.

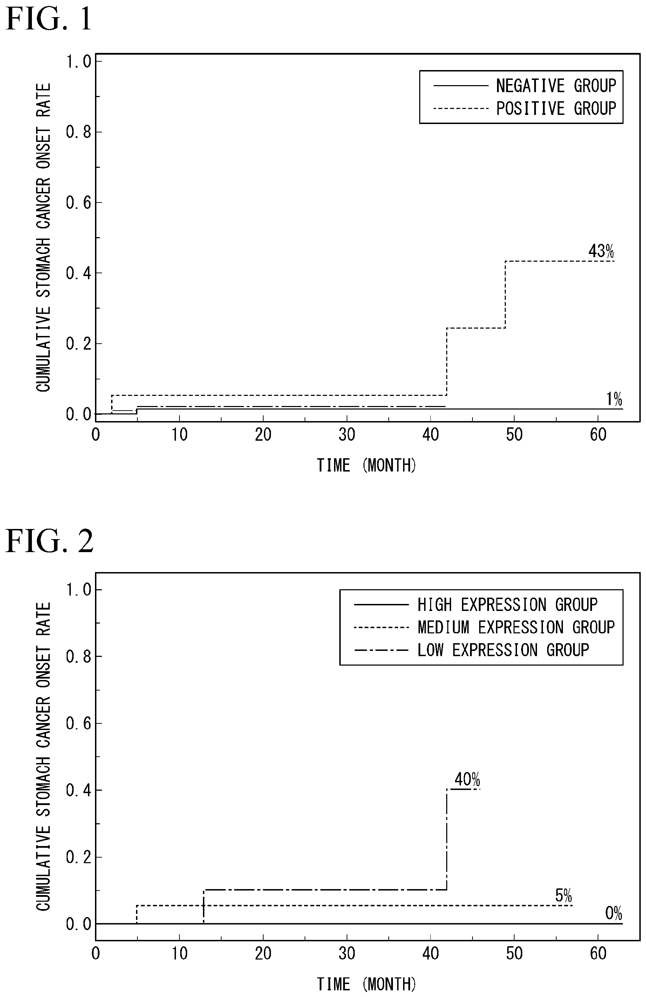

FIG. 1 is a graph showing a 5-year cumulative stomach cancer onset rate in a KK-LC-1 positive group and a negative group. As a result, in the KK-LC-1 positive group, the 5-year cumulative stomach cancer onset rate was 43%. On the other hand, in the KK-LC-1 negative group, the 5-year cumulative stomach cancer onset rate was 1%.

This result indicates that in a case where the expression of the KK-LC-1 gene is positive in the stomach tissue sample of the patient before removing Helicobacter pylori, there is a risk of onset of stomach cancer in the patient after the removal.

### [Experimental Example 2]

### (Study by Real-Time RT-PCR)

In Experimental Example 1, a case of cancer occurrence was also recognized from the KK-LC-1 negative group. Therefore, the detection of the expression of the KK-LC-1 gene was performed using a more sensitive real-time RT-PCR.

In cases of 96 patients after removing Helicobacter pylori, stomach tissue samples collected before the removal were subjected to real-time RT-PCR, and the expression of the KK-LC-1 gene was detected.

In the real-time RT-PCR, a primer consisting of a base sequence set forth in SEQ ID NO: 1 and a primer consisting of a base sequence set forth in SEQ ID NO: 2 were used, and further, a qPCR probe (designated "MGB-D") consisting of a base sequence set forth in SEQ ID NO: 8 was used. MGB-D was a qPCR probe in which a fluorescent substance was modified at the 5' end, and a quencher substance and a minor groove binder (MGB) were modified at the 3' end. MGB increases the Tm value.

The Ct value of β-actin may be used as a reference, and a difference between the Ct value of the KK-LC-1 gene and the Ct value of β-actin was obtained and used as ΔCt. The samples were classified into a KK-LC-1 high expression group, a KK-LC-1 medium expression group, and a KK-LC-1 low expression group based on the ΔCt value. Specifically, a case where ΔCt was -2 or more was defined as the high expression group. In addition, a case where ΔCt was -5 or more and less than -2 was defined as the medium expression group. In addition, a case where ΔCt was less than -5 was defined as the low expression group.

FIG. 2 is a graph showing a 5-year cumulative stomach cancer onset rate in patients in each group. As a result, in the KK-LC-1 high expression group, the 5-year cumulative stomach cancer onset rate was 40%, and in the medium expression group, the 5-year cumulative stomach cancer onset rate was 5%. On the other hand, in the KK-LC-1 low expression group, the 5-year cumulative stomach cancer onset rate was 0%. This result indicates that the detection sensitivity of KK-LC-1 was increased by the highly sensitive real-time RT-PCR. In addition, the result indicates that there is a risk of onset of stomach cancer in a patient after removing Helicobacter pylori, in correlation with the expression amount of the KK-LC-1 gene in the stomach tissue sample of the patient before the removal. In addition, the above result indicates that in a case where the expression amount of the KK-LC-1 gene in the stomach tissue sample of the patient before the removing Helicobacter pylori is low (in a case where ΔCt is less than -5), it can be determined that the risk of onset of stomach cancer in the patient after the removal is low.

### [Experimental Example 3]

### (Study 1 of qPCR Probe)

Using a primer consisting of a base sequence set forth in SEQ ID NO: 1 and a primer consisting of a base sequence set forth in SEQ ID NO: 2, and further using the qPCR probe shown in Table 1, genomic DNA and a plasmid containing cDNA of a KK-LC-1 gene were amplified by real-time PCR. The concentration of the qPCR probe in the reaction solution of the real-time PCR was 0.25 µM.

**[Table 1]**

| Name | SEQ ID NO | Presence or absence of MGB | Number of mutations | Base length |
|---|---|---|---|---|
| MGB-A | 5 | Presence | 4 | 17 |
| MGB-B | 6 | Presence | 3 | 19 |
| MGB-C | 7 | Presence | 2 | 18 |
| MGB-D | 8 | Presence | 2 | 18 |
| Non-MGB-E | 9 | Absence | 2 | 22 |
| Non-MGB-F | 10 | Absence | 1 | 22 |

In Table 1, the presence or absence of the minor groove binder (MGB), the number of mutations, and the base length are shown for each qPCR probe. All the qPCR probes shown in Table 1 had a base sequence including the 195th and 196th bases in the base sequence (SEQ ID NO: 3) of the cDNA of the KK-LC-1 gene. Therefore, all the qPCR probes shown in Table 1 hybridized to the linking site between the first exon and the second exon in the cDNA of the KK-LC-1 gene. In addition, the qPCR probes shown in Table 1 had a one- to four-base mutation with respect to the base sequence of the cDNA of the KK-LC-1 gene (SEQ ID NO: 3). In Table 1, the number of mutations means the number of mutations with respect to SEQ ID NO: 3.

In addition, for comparison, a commercially available qPCR probe (designated "Hs02386421_g1", Thermo Fisher Scientific Inc.) for the cDNA of the KK-LC-1 gene was also used. Hs02386421_g1 was a qPCR probe modified with a fluorescent substance at the 5' end and modified with a quencher substance and MGB at the 3' end. The base sequence of Hs02386421_g1 is not publicly disclosed by the manufacturer, but no mutation is introduced in the base sequence of the cDNA of the KK-LC-1 gene.

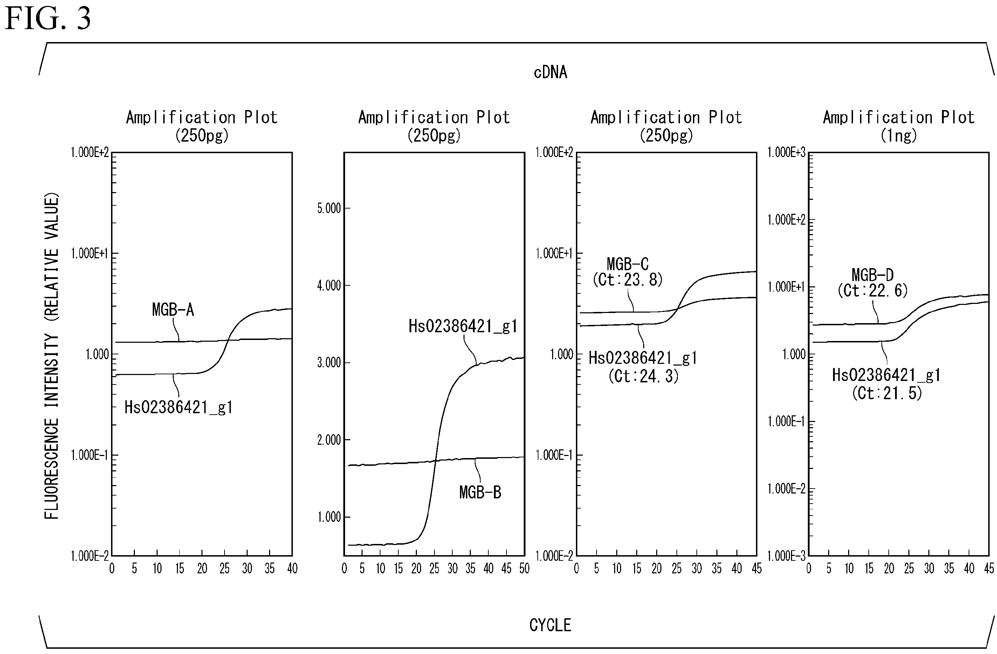

FIG. 3 is a graph showing the results of real-time PCR using a plasmid containing the cDNA of the KK-LC-1 gene as a template. The qPCR probe has to react with the cDNA of the KK-LC-1 gene. In FIG. 3, the vertical axis represents the fluorescence intensity (relative value), and the horizontal axis represents the number of cycles. In addition, the graph shows the amount of the plasmid containing the cDNA of the KK-LC-1 gene used as a template.

As a result, it was shown that MGB-A and MGB-B did not react with the cDNA of the KK-LC-1 gene. On the other hand, MGB-C and MGB-D showed reactivity to the cDNA of the KK-LC-1 gene.

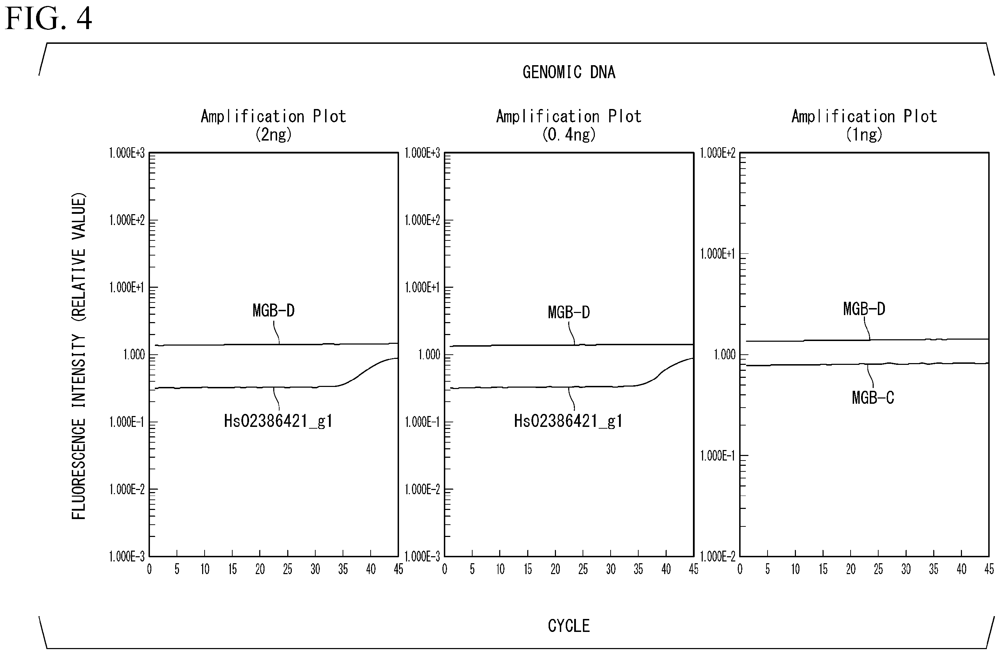

FIG. 4 is a graph showing the results of the real-time PCR using the genomic DNA as a template. In FIG. 4, the vertical axis represents the fluorescence intensity (relative value), and the horizontal axis represents the number of cycles. In addition, the graph shows the amount of genomic DNA used as a template. As the genomic DNA, genomic DNA of H-111-TC, which is a human stomach cancer cell line, was used. It is preferable that the qPCR probe does not react with the genomic DNA.

As a result, it was confirmed that a commercially available qPCR probe (designated "Hs02386421_g1") for the cDNA of the KK-LC-1 gene also reacted with the genomic DNA. On the other hand, it was confirmed that MGB-C and MGB-D did not react with the genomic DNA.

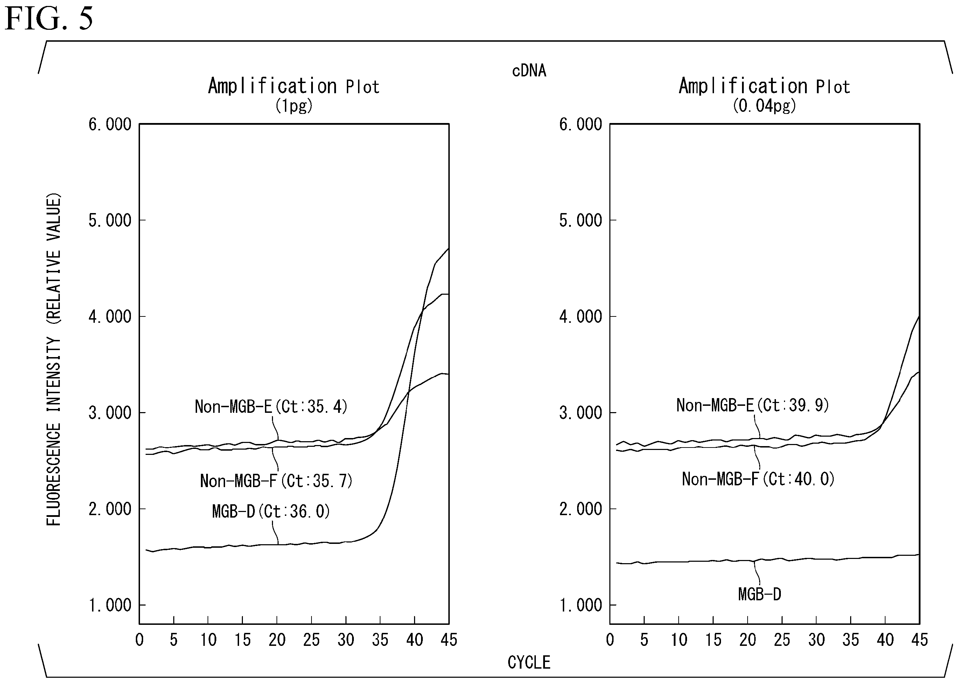

FIG. 5 is a graph showing the results of the real-time PCR using the cDNA as a template. In FIG. 5, the vertical axis represents the fluorescence intensity (relative value), and the horizontal axis represents the number of cycles. In addition, the graph shows the amount of the plasmid containing the cDNA of the KK-LC-1 gene used as a template. The qPCR probe has to react with the cDNA of the KK-LC-1 gene.

As a result, it was shown that Non-MGB-E and Non-MGB-F reacted with the cDNA of the KK-LC-1 gene at the same Ct value as that of MGB-D, from the left side in FIG. 5. In addition, it was shown that Non-MGB-E and Non-MGB-F had higher sensitivity than MGB-D from the right side in FIG. 5.

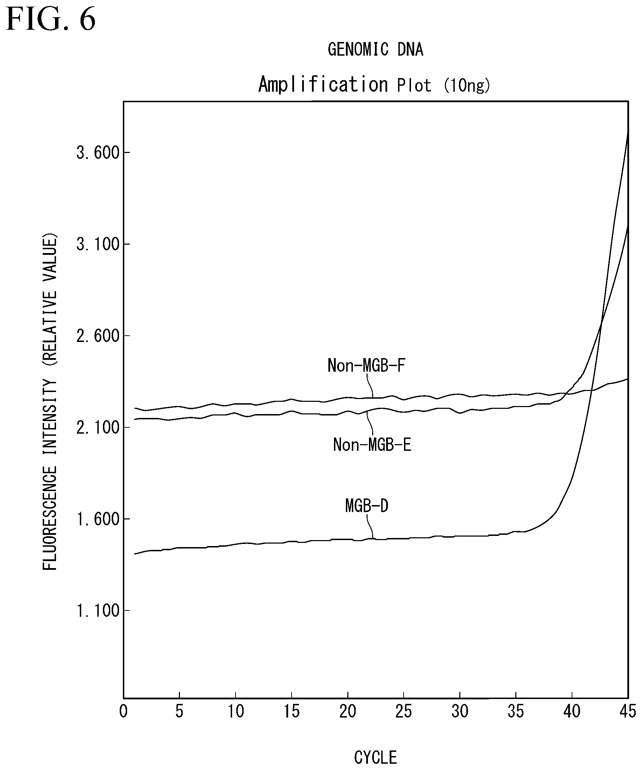

FIG. 6 is a graph showing the results of the real-time PCR using the genomic DNA as a template. In FIG. 6, the vertical axis represents the fluorescence intensity (relative value), and the horizontal axis represents the number of cycles. In addition, the graph shows the amount of genomic DNA used as a template. As the genomic DNA, genomic DNA of H-111-TC, which is a human stomach cancer cell line, was used. It is preferable that the qPCR probe does not react with the genomic DNA.

As a result, it was confirmed that when 10 ng of genomic DNA was used, MGB-D and Non-MGB-E also reacted with the genomic DNA. On the other hand, it was shown that Non-MGB-F had low reactivity to the genomic DNA.

### [Experimental Example 4]

### (Study 2 of qPCR Probe)

Using a primer consisting of a base sequence set forth in SEQ ID NO: 1, a primer consisting of a base sequence set forth in SEQ ID NO: 2, MGB-D, which is the qPCR probe shown in Table 1 above, and a single-stranded nucleic acid fragment shown in Table 2 below, genomic DNA and a plasmid containing cDNA of a KK-LC-1 gene were amplified by real-time PCR. The concentration of the qPCR probe in the reaction solution of the real-time PCR was 0.25 µM. In addition, the concentration of the single-stranded nucleic acid fragment in the real-time PCR reaction solution was 1.0 µM.

**[Table 2]**

| Name | SEQ ID NO | Base length |
|---|---|---|
| Block 1 | 11 | 31 |
| Block 2 | 12 | 31 |
| Block 3 | 13 | 27 |

In Table 2, the base length of each single-stranded nucleic acid fragment is shown. The single-stranded nucleic acid fragments shown in Table 2 suppressed the amplification of the genomic DNA. All of the single-stranded nucleic acid fragments shown in Table 2 had a base sequence including the 322nd and 323rd bases in the base sequence (SEQ ID NO: 4) of the genomic DNA of KK-LC-1. Therefore, all the single-stranded nucleic acid fragments shown in Table 2 hybridized to the linking site between the intron and the second exon in the genomic DNA of KK-LC-1.

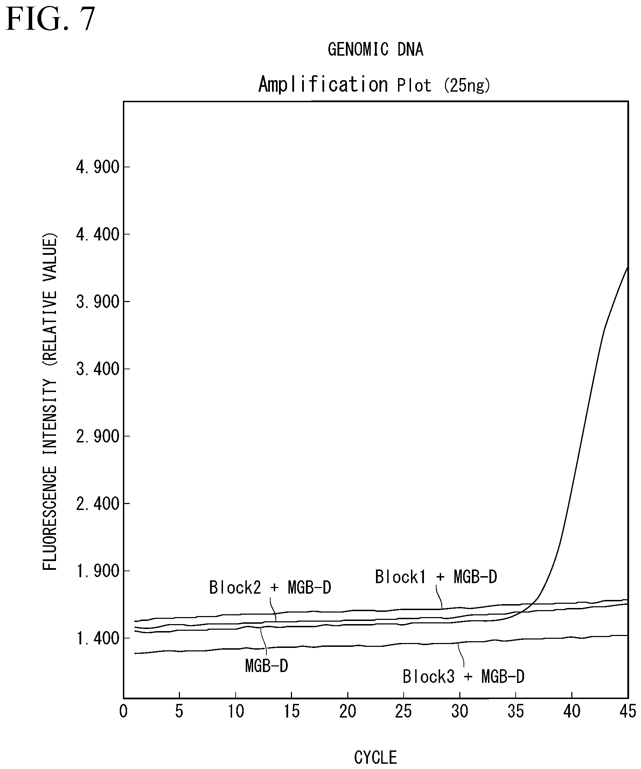

FIG. 7 is a graph showing the results of the real-time PCR using the genomic DNA as a template. In FIG. 7, the vertical axis represents the fluorescence intensity (relative value), and the horizontal axis represents the number of cycles. In addition, the graph shows the amount of genomic DNA used as a template. As the genomic DNA, genomic DNA of H-111-TC, which is a human stomach cancer cell line, was used. It is preferable that the qPCR probe does not react with the genomic DNA.

As a result, it was confirmed that in a case where 25 ng of genomic DNA was used, MGB-D also reacted with the genomic DNA. On the other hand, it was found that in a case where any one of Block 1, Block 2, or Block 3 was allowed to coexist with MGB-D, the reaction of MGB-D to the genomic DNA was suppressed.

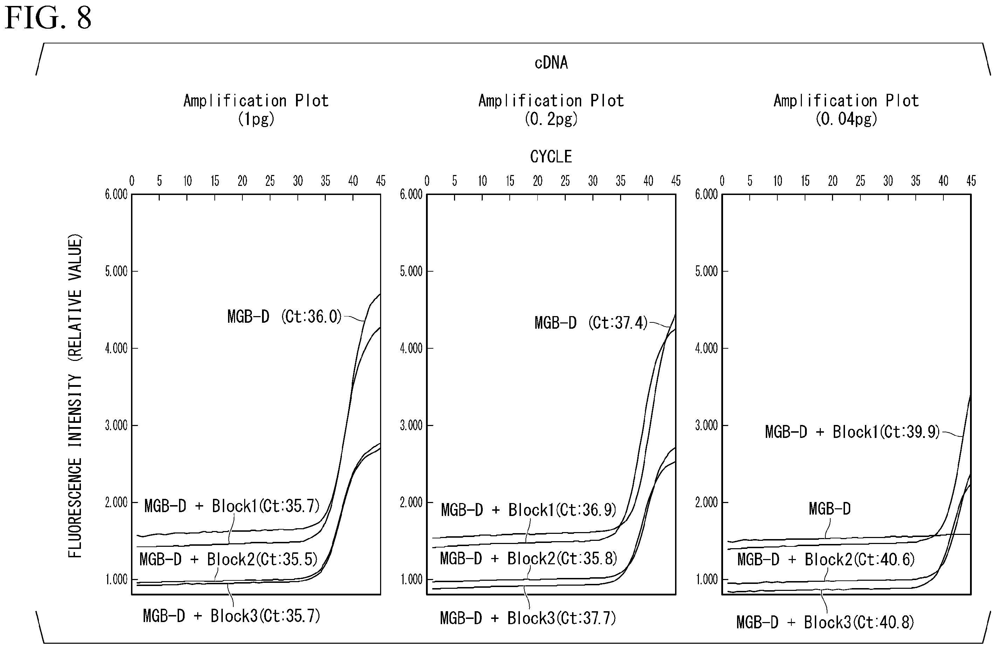

FIG. 8 is a graph showing the results of the real-time PCR using cDNA as a template. In FIG. 8, the vertical axis represents the fluorescence intensity (relative value), and the horizontal axis represents the number of cycles. In addition, the graph shows the amount of the plasmid containing the cDNA of the KK-LC-1 gene used as a template. The qPCR probe has to react with the cDNA of the KK-LC-1 gene.

As a result, it was shown that even when any of Block 1, Block 2, or Block 3 was allowed to coexist with MGB-D, the reactivity of MGB-D was not adversely affected. Rather, as shown on the right side in FIG. 8, it was shown that when any one of Block 1, Block 2, or Block 3 was allowed to coexist with MGB-D, the detection sensitivity of the cDNA of the KK-LC-1 gene was increased.

### [Experimental Example 5]

### (Study 3 of qPCR Probe)

Using a primer consisting of a base sequence set forth in SEQ ID NO: 1, a primer consisting of a base sequence set forth in SEQ ID NO: 2, Non-MGB-E or Non-MGB-F, which is the qPCR probe shown in Table 1 above, and Block 1, which is the single-stranded nucleic acid fragment shown in Table 2 above, genomic DNA and a plasmid containing cDNA of a KK-LC-1 gene was amplified by real-time PCR. The concentration of the qPCR probe in the reaction solution of the real-time PCR was 0.25 µM. In addition, the concentration of the single-stranded nucleic acid fragment in the real-time PCR reaction solution was 1.0 µM. In addition, for comparison, a group in which MGB-D was used as a qPCR probe was also prepared.

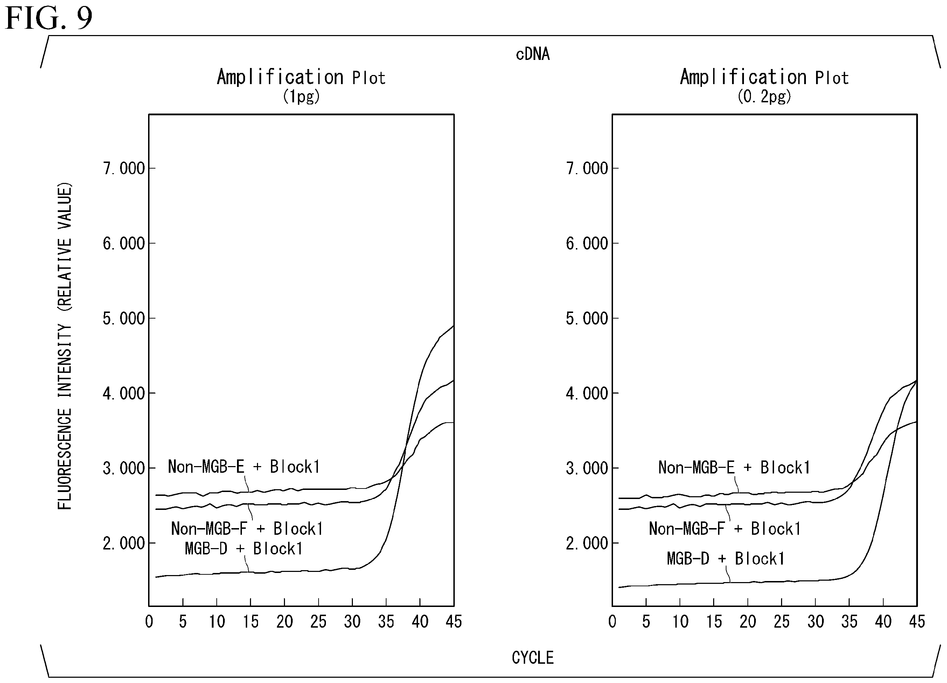

FIG. 9 is a graph showing the results of the real-time PCR using cDNA as a template. In FIG. 9, the vertical axis represents the fluorescence intensity (relative value), and the horizontal axis represents the number of cycles. In addition, the graph shows the amount of the plasmid containing the cDNA of the KK-LC-1 gene used as a template. The qPCR probe has to react with the cDNA of the KK-LC-1 gene.

As a result, it was shown that even when Block 1 was allowed to coexist with Non-MGB-E or Non-MGB-F, the reactivity was not adversely affected.

The above results indicate that the qPCR probe shown in Table 1 above or the combination of the qPCR probe shown in Table 1 above and the single-stranded nucleic acid fragment shown in Table 2 below is suitable for detecting the expression of the KK-LC-1 gene.

### [Experimental Example 6]

### (Study 4 of qPCR probe)

Using a primer consisting of a base sequence set forth in SEQ ID NO: 1, a primer consisting of a base sequence set forth in SEQ ID NO: 2, MGB-D, Non-MGB-E, and Non-MGB-F, which are the qPCR probes shown in Table 1 above, and Block 1, which is the single-stranded nucleic acid fragment shown in Table 2 above, genomic DNA was amplified by real-time PCR.

In addition, for comparison, a commercially available qPCR probe (designated "Hs02386421_g1", Thermo Fisher Scientific Inc.) for the cDNA of the KK-LC-1 gene was also used. Hs02386421_g1 was a qPCR probe modified with a fluorescent substance at the 5' end and modified with a quencher substance and MGB at the 3' end. The base sequence of Hs02386421_g1 is not publicly disclosed by the manufacturer, but no mutation is introduced in the base sequence of the cDNA of the KK-LC-1 gene.

The concentration of the qPCR probe in the reaction solution of the real-time PCR was 0.25 µM. In addition, the concentration of the single-stranded nucleic acid fragment in the real-time PCR reaction solution was 1.0 µM.

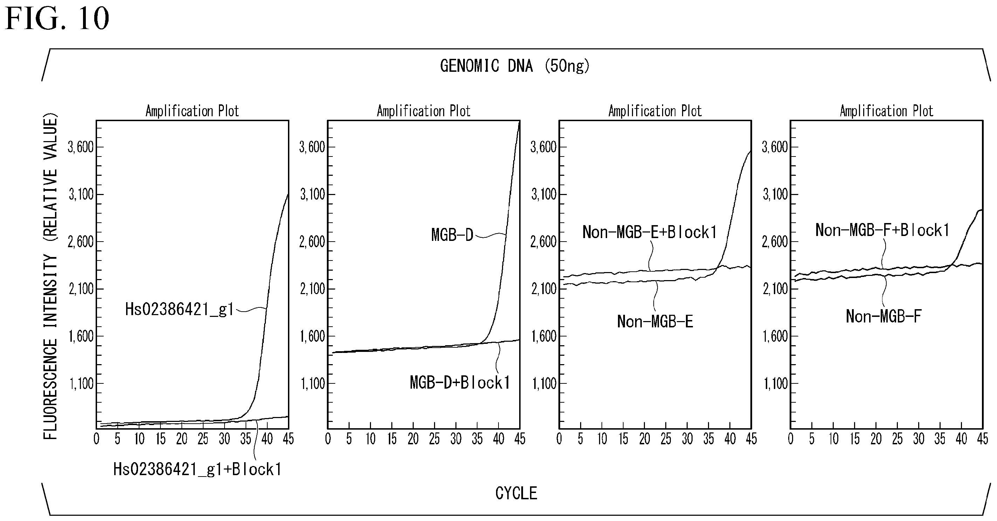

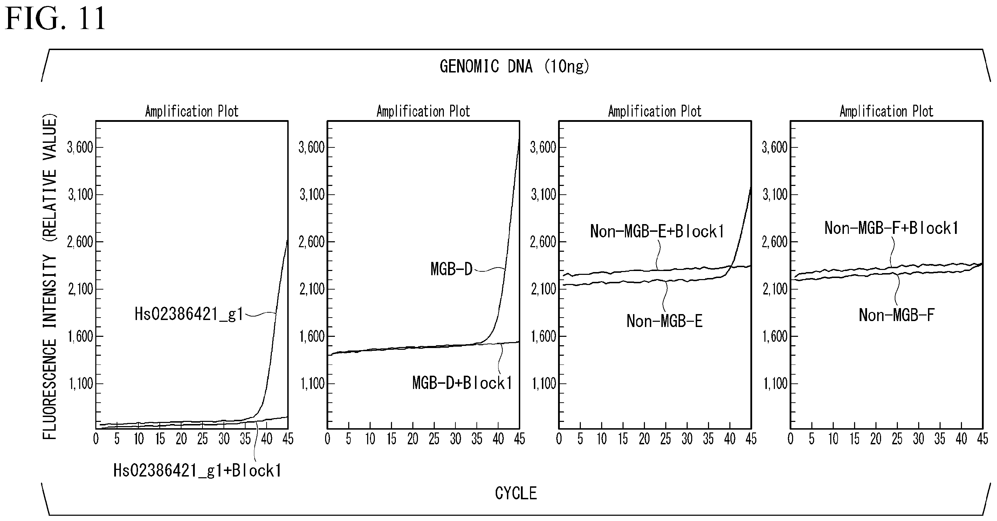

FIGS. 10 and 11 are graphs showing the results of the real-time PCR using genomic DNA as a template. In FIGS. 10 and 11, the vertical axis represents the fluorescence intensity (relative value), and the horizontal axis represents the number of cycles. In addition, the graph shows the amount of genomic DNA used as a template. As the genomic DNA, genomic DNA of H-111-TC, which is a human stomach cancer cell line, was used. It is preferable that the qPCR probe does not react with the genomic DNA.

As a result, it was confirmed that Hs02386421_g1, MGB-D, Non-MGBE, and Non-MGB-F also reacted with the genomic DNA. On the other hand, it was found that when Block 1 was allowed to coexist with these qPCR probes, the reaction to the genomic DNA was suppressed.

### [Experimental Example 7]

### (Study Using Clinical Specimens)

Cancer tissue samples derived from breast cancer patients and cancer tissue samples derived from colon cancer patients were each subjected to end-point RT-PCR and real-time RT-PCR and the expression of the KK-LC-1 gene was detected.

In the end-point RT-PCR, a primer consisting of a base sequence set forth in SEQ ID NO: 1 and a primer consisting of a base sequence set forth in SEQ ID NO: 2 were used. After 40 cycles of PCR reaction, agarose gel electrophoresis was performed, a case where a visible band was detected was determined to be positive, and a case where a visible band was not detected was determined to be negative.

In the real-time RT-PCR, a primer consisting of a base sequence set forth in SEQ ID NO: 1 and a primer consisting of a base sequence set forth in SEQ ID NO: 2, and MGB-D, which is the qPCR probe shown in Table 1 above, were used. In addition, the real-time RT-PCR was performed in the presence or absence of Block 1, which is the single-stranded nucleic acid fragment shown in Table 2 above. The Ct value of β-actin may be used as a reference, and a difference between the Ct value of the KK-LC-1 gene and the Ct value of β-actin was obtained and used as ΔCt.

The results of using the cancer tissue samples derived from the breast cancer patients are shown in Table 3 below, and the results of using the cancer tissue samples derived from the colon cancer patients are shown in Table 4 below. As a result, even in a case where the result was below the detection limit in the end-point RT-PCR, the expression of the KK-LC-1 gene could be detected in the real-time RT-PCR, except for the specimen number 7 derived from the colon cancer patient. In the specimen number 7 derived from the colon cancer patient, the expression of the KK-LLC-1 gene was not detected in both the end-point RT-PCR and the real-time RT-PCR.

In the specimen numbers 8 and 9 derived from the breast cancer patients and the specimen numbers 3, 5, 6, and 8 derived from the colon cancer patients, the expression of the KK-LC-1 gene was detected in the absence of Block 1, but the expression of the KK-LC-1 gene was not detected in the presence of Block 1. This result was considered to be a false positive result in which the qPCR probe reacted with the genomic DNA in the absence of Block 1. In the presence of Block 1, it was shown that false positivity was suppressed and accurate expression of the KK-LC-1 gene was detected.

**[Table 3]**

| (Breast cancer) | | | | | |
|---|---|---|---|---|---|
| Specimen number | Result of end-point RT-PCR | Condition for real-time RT-PCR | Ct value | Ct value of β-actin | ΔCt |
| 1 | Below detection limit | MGB-D | 38.3 | 25.1 | -13.2 |
| 1 | Below detection limit | MGB-D + Block 1 | 41.5 | 25.1 | -16.4 |
| 2 | Below detection limit | MGB-D | 32.3 | 23.5 | -8.8 |
| 2 | Below detection limit | MGB-D + Block 1 | 32.4 | 23.5 | -8.9 |
| 3 | Below detection limit | MGB-D | 34.5 | 26.6 | -7.9 |
| 3 | Below detection limit | MGB-D + Block 1 | 34.8 | 26.6 | -8.2 |
| 4 | Below detection limit | MGB-D | 32.1 | 23.7 | -8.4 |
| 4 | Below detection limit | MGB-D + Block 1 | 32.2 | 23.7 | -8.5 |
| 5 | Below detection limit | MGB-D | 33.5 | 24.5 | -9.0 |
| 5 | Below detection limit | MGB-D + Block 1 | 34.3 | 24.5 | -9.8 |
| 6 | Below detection limit | MGB-D | 34.2 | 23.4 | -10.8 |
| 6 | Below detection limit | MGB-D + Block 1 | 36.8 | 23.4 | -13.4 |
| 7 | Below detection limit | MGB-D | 35.4 | 24.7 | -10.7 |
| 7 | Below detection limit | MGB-D + Block 1 | 35.2 | 24.7 | -10.5 |
| 8 | Below detection limit | MGB-D | 36.8 | 27.0 | -9.8 |
| 8 | Below detection limit | MGB-D + Block 1 | Below detection limit | 27.0 | Uncountable |
| 9 | Below detection limit | MGB-D | 36.4 | 27.9 | -8.5 |
| 9 | Below detection limit | MGB-D + Block 1 | Below detection limit | 27.9 | Uncountable |
| 10 | Below detection limit | MGB-D | 35.9 | 25.4 | -10.5 |
| 10 | Below detection limit | MGB-D + Block 1 | 37.4 | 25.4 | -12.0 |

**[Table 4]**

| (Colon cancer) | | | | | |
|---|---|---|---|---|---|
| Specimen number | Result of end-point RT-PCR | Condition for real-time RT-PCR | Ct value | Ct value of β-actin | ΔCt |
| 1 | Below detection limit | MGB-D | 37.2 | 25.9 | -11.3 |
| 1 | Below detection limit | MGB-D + Block 1 | 38.5 | 25.9 | -11.3 |
| 2 | Below detection limit | MGB-D | 39.0 | 26.5 | -12.5 |
| 2 | Below detection limit | MGB-D + Block 1 | 39.0 | 26.5 | -12.5 |
| 3 | Below detection limit | MGB-D | 39.2 | 26.8 | -12.4 |
| 3 | Below detection limit | MGB-D + Block 1 | Below detection limit | 26.8 | Uncountable |
| 4 | Below detection limit | MGB-D | 38.8 | 25.6 | -13.2 |
| 4 | Below detection limit | MGB-D + Block 1 | 40.9 | 25.6 | -15.3 |
| 5 | Below detection limit | MGB-D | 38.8 | 25.6 | -13.2 |
| 5 | Below detection limit | MGB-D + Block 1 | Below detection limit | 25.6 | Uncountable |
| 6 | Below detection limit | MGB-D | 42.0 | 26.4 | -15.6 |
| 6 | Below detection limit | MGB-D + Block 1 | Below detection limit | 26.4 | Uncountable |
| 7 | Below detection limit | MGB-D | Below detection limit | 27.1 | Uncountable |
| 7 | Below detection limit | MGB-D + Block 1 | Below detection limit | 27.1 | Uncountable |
| 8 | Below detection limit | MGB-D | 35.4 | 27.3 | -8.1 |
| 8 | Below detection limit | MGB-D + Block 1 | Below detection limit | 27.3 | Uncountable |
| 9 | Below detection limit | MGB-D | 33.5 | 27.7 | -5.8 |
| 9 | Below detection limit | MGB-D + Block 1 | 33.2 | 27.7 | -5.5 |
| 10 | Below detection limit | MGB-D | 33.5 | 25.5 | -8.0 |
| 10 | Below detection limit | MGB-D + Block 1 | 33.7 | 25.5 | -8.2 |

### Industrial Applicability

According to the present invention, a technology for assessing a risk of onset of stomach cancer in a patient after removing Helicobacter pylori can be provided.

## Claims

1. A method for assessing a risk of onset of stomach cancer in a patient after removing Helicobacter pylori, the method comprising:
a step of detecting expression of a KK-LC-1 gene in a stomach tissue sample derived from the patient before removing Helicobacter pylori,
wherein positive expression of the KK-LC-1 gene indicates that there is a risk of onset of stomach cancer in the patient after removing Helicobacter pylori.

2. The method according to claim 1,
wherein the detecting of the expression of the KK-LC-1 gene is performed by end-point RT-PCR.

3. The method according to claim 2,
wherein in the end-point RT-PCR, a primer consisting of a base sequence set forth in SEQ ID NO: 1 and a primer consisting of a base sequence set forth in SEQ ID NO: 2 are used.

4. The method according to claim 1,
wherein the detecting of the expression of the KK-LC-1 gene is performed by real-time RT-PCR.

5. The method according to claim 4,
wherein in the real-time RT-PCR, a qPCR probe is used, and
the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.

6. The method according to claim 4 or 5,
wherein the real-time RT-PCR is performed in presence of a single-stranded nucleic acid fragment that suppresses amplification of genomic DNA, and
the single-stranded nucleic acid fragment has a base sequence including 322nd and 323rd bases or 1096th and 1097th bases in a base sequence set forth in SEQ ID NO: 4, and has a base length of 20 to 50 bases.

7. The method according to claim 1,
wherein the detecting of the expression of the KK-LC-1 gene is performed at a protein level.

8. A kit for assessing a risk of onset of stomach cancer in a patient after removing Helicobacter pylori, the kit comprising:
a primer consisting of a base sequence set forth in SEQ ID NO: 1; and
a primer consisting of a base sequence set forth in SEQ ID NO: 2.

9. The kit according to claim 8, further comprising:
a qPCR probe,
wherein the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.

10. The kit according to claim 8 or 9, further comprising:
a single-stranded nucleic acid fragment that suppresses amplification of genomic DNA,
wherein the single-stranded nucleic acid fragment has a base sequence including 322nd and 323rd bases or 1096th and 1097th bases in a base sequence set forth in SEQ ID NO: 4, and has a base length of 20 to 50 bases.

11. A kit for assessing a risk of onset of stomach cancer in a patient after removing Helicobacter pylori, the kit comprising:
a specific binding substance to a KK-LC-1 protein.

12. A polynucleotide having a base sequence including 195th and 196th bases in a base sequence set forth in SEQ ID NO: 3, having a base length of 15 to 50 bases, and having a one- or two-base mutation with respect to the base sequence set forth in SEQ ID NO: 3.

13. A polynucleotide having a base sequence including 322nd and 323rd bases or 1096th and 1097th bases in a base sequence set forth in SEQ ID NO: 4, and having a base length of 20 to 50 bases.

14. A method for detecting expression of a KK-LC-1 gene in a biological sample,
wherein the detecting is performed by real-time RT-PCR,
in the real-time RT-PCR, a qPCR probe is used,
the real-time RT-PCR is performed in presence of a single-stranded nucleic acid fragment that suppresses amplification of genomic DNA, and
the single-stranded nucleic acid fragment has a base sequence including 322nd and 323rd bases or 1096th and 1097th bases in a base sequence set forth in SEQ ID NO: 4, and has a base length of 20 to 50 bases.

15. The method according to claim 14,
wherein the qPCR probe has a base sequence including 195th and 196th bases in a base sequence set forth in SEQ ID NO: 3, has a base length of 15 to 50 bases, and has a one- or two-base mutation with respect to the base sequence set forth in SEQ ID NO: 3.
